# EUROPEAN PATENT APPLICATION

(11) **EP 1 082 948 A1**
(43) Date of publication of application: **14.03.2001**
(21) Application number: 00119514.8
(22) Date of filing: 07.09.2000
(51) Int. Cl.: A61F 2/30, A61F 2/36

(54) **Device to center a femoral shaft**

(30) Priority: 09.09.1999 IT UD990165
(71) Applicant: LIMA Lto SpA, 33030 Villanova di S. Daniele (UD) (IT)
(72) Inventor: Lualdi, Gabriele, 33034 FAGAGNA(UD) (IT)
(74) Representative: Petraz, Gilberto Luigi

(57) **Abstract**

Device to center a femoral shaft (10) and relative centering method, wherein the femoral shaft (10) comprises a distal part (12) able to be inserted into the diaphyseal canal (13) of a femur (11) and a proximal part (14) with which a corresponding femoral head (16) is able to be coupled. The device comprises a first axial guide element (20) able to be coupled both with a diaphyseal element (22) which is inserted and clamped in said diaphyseal canal (13) of the femur (11) and with the distal part (12) of the femoral shaft (10) to center at least such distal part (12) with respect to the diaphyseal canal (13) of the femur (11).

## Description

### FIELD OF THE INVENTION

This invention refers to a device and the relative method to center a femoral shaft, to position it simply and accurately with respect to the femur into which it is inserted as a prosthesis, to support an artificial femoral head.

More in particular, the device comprises two guide elements, preferably made of plastic material, i.e. a distal element to axially position the corresponding distal part of the femoral shaft inside the diaphyseal canal, and a proximal element, which transversely positions the corresponding proximal part of the said femoral shaft.

### BACKGROUND OF THE INVENTION

In order to position a femoral shaft inside a femur, the state of the art normally teaches to make a seating in the proximal part of the diaphyseal canal by removing material; the seating should possibly have the same size as the proximal part of the shaft. The seating is made using the appropriate instruments during the surgical operation to install the prosthesis. This takes a long time and a great deal of accuracy, so that the seating is the same size as the part of the femoral shaft which it has to couple with; the results are not always of the best, and this has deleterious consequences on the position and angle of the shaft with respect to the femur.

The state of the art also teaches that to prevent the shaft from penetrating excessively into the diaphyseal canal, the shaft is provided with a transverse stop collar, able to collaborate with the plane on which the resection is made on the neck of the femur.

In the state of the art diaphyseal plugs are also used, which prevent the fixing cement from going too far down inside the diaphyseal canal.

The following documents disclose different femoral shaft: FR-A-2,662,931; US-A-5,766,262; DE-A-4313201; EP-A-0379785; US-A-4,994,085; WO-A-97/27828; FR-A-2,325,355; WO-A-97/40785; WO-A-93/01773.

Therefore, techniques which have been used so far have tried to solve the problem of centering the femoral shafts either by making positioning and stopping elements, of a single piece with the shafts themselves, or by modelling and shaping the femur seating able to accommodate the shaft, with the disadvantage that the operation to insert the shaft into the femur is long and complex, with negative consequences both for the patient, who has to stay anaesthetized for a long time, and also for the economics of the operation, considering the costs of the medical and auxiliary staff.

The present Applicant has devised, tested and embodied the device and method according to the invention to overcome the shortcomings described above and to perfect the known devices and methods to center femoral shafts.

### SUMMARY OF THE INVENTION

The device and method to center a femoral shaft according to the invention are set forth and characterized in the main claims, while the dependent claims describe other innovative characteristics of the invention.

One purpose of the invention is to achieve a device which makes possible to center, precisely, simply and reliably, both in the distal zone and in the proximal zone, a femoral shaft inserted inside a femur which needs the relative head replacing.

In accordance with this purpose, the centering device for a femoral shaft according to the invention comprises a first axial guide element coupled both with a diaphyseal element able to be inserted and fixed in the corresponding diaphyseal canal of the femur and with the distal part of the femoral shaft to center such a distal part with respect to the diaphyseal canal of the femur.

The diaphyseal element comprises a substantially cylindrical plug having an inner cavity, while the first axial guide element comprises a substantially cylindrical cup having the peripheral wall able to be coupled with precision with the inner cavity of the diaphyseal element and an internal cavity able to be coupled with precision with the distal part of the femoral shaft.

According to another characteristic of the invention, a second proximal guide element, comprising a guide through cavity, is able to be attached to the proximal meta-epiphysis of the femur to guide, by means of its guide through cavity, the proximal part of the femoral shaft and center it transversely with respect to the femur.

According to another characteristic of the invention, the second proximal guide element comprises side walls which define the through cavity and an upper collar able to rest on the resection plane of the proximal epiphysis of the femur.

According to another characteristic of the invention, the method to center the femoral shaft comprises a first step of inserting and clamping a diaphyseal element in the diaphyseal canal, a second step of coupling the distal part of the femoral shaft with an internal cavity of a first axial guide element to perform a precise coupling thereof, and a third step of inserting the femoral shaft and the first axial guide element coupled thereto into the diaphyseal canal of the femur until the peripheral wall of said first axial guide element is inserted with precision into said inner cavity of the diaphyseal element.

### BRIEF DESCRIPTION OF THE DRAWINGS

These and other characteristics of the invention will become clear from the following description of a preferred form of embodiment, given as a non-restrictive example with reference to the attached drawings wherein:
- Fig. 1: is a schematic side view of a femoral shaft associated with a femur, into which it is able to be inserted as a prosthesis;
- Fig. 2a: is an enlarged and schematic side view of the femoral shaft as in Fig. 1, associated with the centering device according to the invention;
- Fig. 2b: is a partial schematic side view of the femoral shaft as in Fig. 2 with some parts disassembled;
- Fig. 3: is a side view of the proximal guide element of the centering device according to the invention; and
- Fig. 4: is a view from above of the guide element in Fig. 3.

### DETAILED DESCRIPTION OF PREFERRED EMBODIMENT

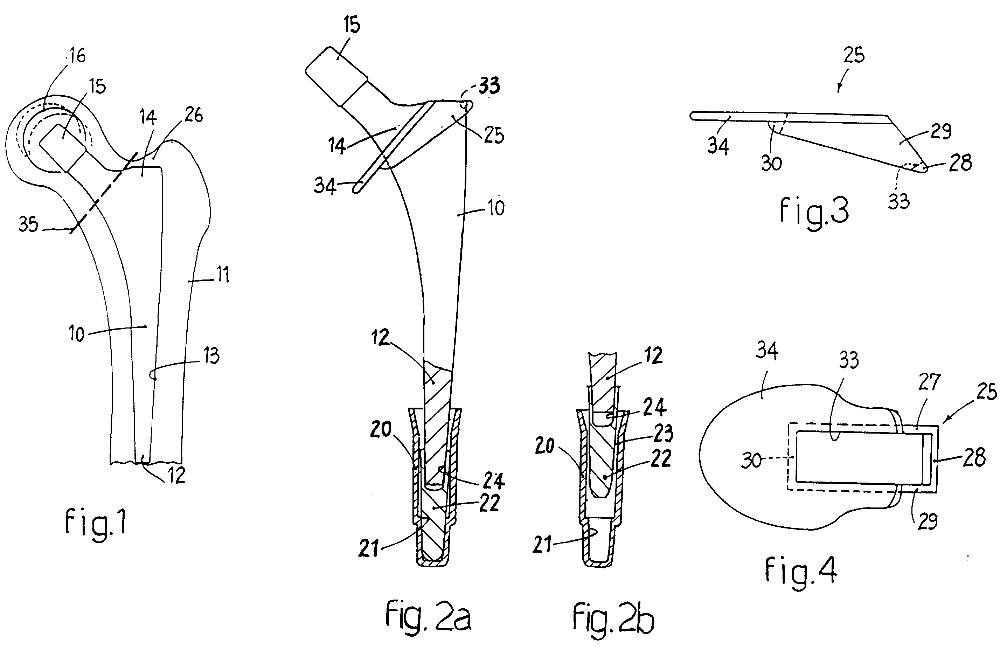

With reference to Figs. 1-4, a femoral shaft 10 is shown associated with a femur 11 into which it is able to be inserted as a prosthesis.

The femoral shaft 10 is made of resistant metal, such as for example steel or non-ferrous alloy, and comprises a distal part 12, able to be inserted into the diaphyseal canal 13 of the femur 11, and a proximal part 14 provided with a tapered end 15, able to be coupled with a corresponding femoral head 16.

The device according to the present invention comprises a diaphyseal element constituted by a substantially cylindrical plug 20 able to be inserted and fixed in the diaphyseal canal 13 of the femur 11 and provided with an inner cavity 21. A first axial guide element, constituted by a substantially cylindrical or slightly tapered cup 22, has the peripheral wall 23 able to be coupled with precision with the inner cavity 21 of the plug 20 and an internal cavity 24 able to be coupled with precision the distal part 12 of the femoral shaft 10.

The plug 20 ad the cup 22 may be made either of rubber or of plastic material, such as for example polyethylene, polymethyl methacrylate or any other resorbable material.

The centering device according to the present invention also comprises a second proximal guide element or plug 25, which is able to be coupled to the proximal epiphysis 26 of the femur 11 to cooperate with the proximal part 14 of the femoral shaft 10 and to center it transversely with respect to the femur 11.

The proximal guide element 25 is also made of plastic material, such as for example polyethylene or polymethyl methacrylate, and comprises side walls 27, 28, 29 and 30 which define a through cavity 33, with a substantially rectangular cross section, inside which the proximal part 14 of the femoral shaft 10 is able to be inserted and precisely guided.

The element 25 also comprises an upper collar 34, able to rest on the resection plane 35 of the proximal epiphysis 26 of the femur 11.

The walls 27 and 29 are substantially parallel to each other, while the walls 28 and 30 are slightly convergent downwards, and therefore the longitudinal section of the cavity 33 is substantially shaped like a truncated cone, to facilitate the centering of the femoral shaft 10.

The through cavity 33 may be either centered or misaligned with respect to the upper collar 34.

After the neck of the femur 11 has been cut and the resection plane 35 has thus been defined, the femoral shaft 10 is inserted into the femur 11 and centered by the device according to the invention in the following manner:

The femoral epiphysis is perforated using a suitable blade and the diaphyseal canal 13 is then widened, using reamers which increase in diameter until the endosteum cortex. The diameter of the last reamer used will correspond to the diameter of the diaphyseal plug 20 to be used.

The last reamer is left in situ, and only the handle is removed.

Using the shaft of the reamer as a vertical guide, a trocantheric seating is made, first by means of a punch and then by means of rasps, with a substantially rectangular cross section and suitable to accommodate the part of the proximal plug 25 below the collar 34 with great precision.

When the seating for the shaft 10 and the relative proximal plug 25 has been completed, and the size of the femoral head 16 to be installed has been decided upon, both the reamer and the last rasp used are removed from the femur 11.

The diaphyseal plug 20 is introduced into the diaphyseal canal, using a conventional introduction element.

Then the diaphyseal canal 13 is filled with a suitable cement.

The proximal plug 25 is inserted into the seating made with the rasp and is maintained therein.

The distal part 12 of the femoral shaft 10 is inserted into the internal cavity 24 of the cup 22 to be precisely coupled thereto.

The femoral shaft 10 and the cup 22 coupled thereto are introduced from above into the through cavity 33 of the proximal plug 25, normally of a size corresponding to the last rasp used, until the proximal part 14 reaches the correct level with respect to the corresponding through cavity 33 of the proximal plug 25. At the same time the cup 22 reaches the lower part of the inner cavity 21 of the plug 20 and realizes a precise coupling therewith.

The presence of the proximal plug 25 lodged with precision into the seating of the femoral epiphysis allows the cement inserted into the diaphyseal canal 13 to be compressed by the same femoral shaft 10 during the introduction thereof into the diaphyseal canal 13.

At the end of this step, the cement which has leaked from the diaphyseal canal 13 is removed.

In this way, the shaft 10 is perfectly centered in the femur 11, both axially, thanks to the axial guide element 22, and also transversely, thanks to the proximal guide element 25.

It is obvious that modifications and additions may be made to the device and method to center a femoral shaft as described heretofore, but these shall remain within the field and scope of the invention.

It is also obvious that, although the invention has been described with reference to specific examples, a skilled person in the art shall certainly be able to achieve many other equivalent forms of the device and method, but these shall all come within the field and scope of this invention.

## Claims

1. Device to center a femoral shaft (10) able to be attached in the proximal part of a femur (11), wherein said femoral shaft (10) comprises a distal part (12) able to be inserted into the diaphyseal canal (13) of said femur (11) and a proximal part (14) with which a corresponding femoral head (16) is able to be coupled, characterized in that a first axial guide element (22) is provided to be coupled both with a diaphyseal element (20) able to be inserted and clamped in the corresponding diaphyseal canal (13) of the femur (11) and with the distal part (12) of said femoral shaft (10) to center said distal part (12) with respect to said diaphyseal canal (13) of the femur (11).

2. Centering device as in claim 1, characterized in that said diaphyseal element comprises a substantially cylindrical plug (20) having an inner cavity (21) and in that said first axial guide element comprises a substantially cylindrical cup (22) having the peripheral wall (23) able to be coupled with precision with said inner cavity (21) of said diaphyseal element (20) and an internal cavity (24) able to be coupled with precision with said distal part (12) of said femoral shaft (10).

3. Centering device as in claim 1, characterized in that a second proximal guide element (25) is able to be attached to the proximal resection (26) of said femur (11) to cooperate with said proximal part (14) of said femoral shaft (10) to center it transverse with respect to said femur (11).

4. Centering device as in claim 3, characterized in that said second proximal guide element (25) comprises a through cavity (33) mating with said proximal part (14) of said femoral shaft (10).

5. Centering device as in claim 4, characterized in that said first axial guide element (20) is able to be arranged inside said through cavity (33).

6. Centering device as in Claim 3, characterized in that said second proximal guide element (25) comprises side walls (27, 28, 29, 30) which define said through cavity (33) and an upper collar (34) able to rest on the resection plane (35) of the proximal meta-epiphysis (26) of said femur (11).

7. Centering device as in Claim 6, characterized in that two of said walls (27, 29) are substantially parallel, while two other walls (28, 30) slightly converge downwards, so that the longitudinal section of said through cavity (33) is substantially like a truncated cone, to couple precisely with the mating proximal part (14) of said femoral shaft (10) and achieve the precise centering of the latter with respect to said femur (11).

8. Centering device as in Claim 2, characterized in that said first axial guide element (22) is made of metal, polyethylene or polymethyl methacrylate.
